# EUROPÄISCHE PATENTSCHRIFT

(11) **EP 3 511 025 B1**
(45) Veröffentlichungstag und Bekanntmachung des Hinweises auf die Patenterteilung: **22.06.2022**
(21) Anmeldenummer: 18151208.8
(22) Anmeldetag: 11.01.2018
(51) Int. Cl.: A61L 2/18, A61B 90/70, A61L 2/24, A47L 15/48

(54) **REINIGUNGS- UND DESINFEKTIONSGERÄT SOWIE VERFAHREN ZUM TROCKNEN MEDIZINISCHER UND/ODER ZAHNMEDIZINISCHER INSTRUMENTE**
CLEANING AND DISINFECTING DEVICE AND METHOD FOR DRYING MEDICAL AND/OR DENTAL INSTRUMENTS
APPAREIL DE NETTOYAGE ET DE DÉSINFECTION AINSI QUE PROCÉDÉ DE SÉCHAGE DES INSTRUMENTS MÉDICAUX ET/OU MÉDICODENTAIRES

(43) Veröffentlichungstag der Anmeldung: 17.07.2019
(73) Patentinhaber: MELAG Medizintechnik GmbH & Co. KG, 10829 Berlin (DE)
(72) Erfinder: HÖRNING, Andreas, 10969 Berlin (DE); ZIEBART, Eric, 16321 Bernau (DE); MÖLLER, Janis, 22310 Hamburg (DE); LITZBA, Guido, 14513 Teltow (DE)
(74) Vertreter: Maikowski & Ninnemann Patentanwälte Partnerschaft mbB

(56) Entgegenhaltungen:
- EP-A1- 2 641 619
- CN-A- 106 151 592
- JP-B2- 3 242 310
- US-A- 3 807 420
- US-A- 4 470 429

## Beschreibung

Die vorliegende Erfindung betrifft ein Reinigungs- und Desinfektionsgerät für medizinische und/oder zahnmedizinische Instrumente gemäß dem Oberbegriff des Anspruchs 1 sowie ein Verfahren zum Trocknen medizinischer und/oder zahnmedizinischer Instrumenten gemäß dem Oberbegriff des Anspruchs 10.

EP 2 641 619 A1 offenbart einen Reinigungs- und Desinfektionsautomat für medizinische Instrumente und Geräte, mit einem Spülbottich, der einen mit einer Tür fluiddicht verschließbaren Spülraum bereitstellt und mit einer im Spülraum angeordneten Fluidabgabeeinrichtung die der Abgabe von Spülflotte einerseits und von Trocknungsluft andererseits dient. Um eine alternative Konstruktion eines Reinigungs-und Desinfektionsautomaten bereitzustellen, die eine verbesserte Handhabung gestaltet, wird mit der Erfindung ein Reinigungs- und Desinfektionsautomat vorgeschlagen mit einer außerhalb des Spülraums ausgebildeten Trenn- und Verteileinrichtung zur Beschickung der Fluidabgabeeinrichtung entweder mit Spülflotte oder mit Trocknungsluft, wobei die Trenn- und Verteileinrichtung eingangsseitig einen Spülflottenanschluss und einen davon getrennt ausgebildeten Trocknungsluftanschluss sowie ausgangsseitig einen gemeinsamen Ausgangsanschluss für Spülflotte einerseits und Trocknungsluft andererseits auf.

US 4 470 429 A offenbart ein Dreiwege-Drehkegelventil, das mittels eines elektrischen Stelltriebs geschaltet werden kann, so dass sowohl die Ein- und Auslässe selektiv geöffnet und geschlossen werden können.

Reinigungs- und Desinfektionsgeräte mit aktiver Trocknung weisen in der Regel ein Heißluftgebläse auf, mit dem Trocknungsluft auf zuvor gereinigte und desinfizierte Gegenstände geleitet wird. Diese Trocknungsluft dient der Beschleunigung der Trocknung bzw. der Reduzierung der Restfeuchtigkeit. Dabei wird grundsätzlich zwischen der Innentrocknung und der Außentrocknung der zu trocknenden Gegenstände unterschieden.

Bei der Innentrocknung wird die Trocknungsluft durch ein Innenlumen eines zu trocknenden Instrumentes geleitet. Dazu ist das entsprechende Instrument in der Regel über einen Adapter mit einer Leitung verbunden, über die Trocknungsluft zum Innenlumen des Instruments geführt wird. Die Trocknung wird durch die aus der thermischen Desinfektion der Instrumente resultierende Eigenwärme der Instrumente erreicht und durch die durch die Instrumente durchgeleitete Trocknungsluft beschleunigt. Dabei wird in der Regel eine schnellstmögliche Trocknung angestrebt.

Bei der Außentrocknung der Instrumente strömt die Trocknungsluft in die Reinigungskammer des Reinigungs- und Desinfektionsgeräts und unterstützt die Trocknung der Außenflächen der Instrumente, die teilweise ebenfalls durch die Eigenwärme der Instrumente erreicht wird.

Aus dem Stand der Technik sind verschiedene Konzepte bekannt, wie Trocknungsluft zu Innenbereichen und Außenbereichen von zu trocknenden Gegenständen in einem Reinigungs- und Desinfektionsgerät geleitet werden kann.

Die WO 2006/127478 A1 beschreibt eine Geschirrspülmaschine mit einer Tür, bei der Trocknungsluft aus der Umgebung durch einen unteren Bereich der Tür angesaugt und durch einen oberen Bereich der Tür wieder in die Umgebung abgegeben wird. Eine spezielle Trennung zwischen Luft auf der einen Seite und Spülflüssigkeit auf der anderen Seite ist dabei nicht vorgesehen.

Aus der DE 10 2014 222 541 A1 ist eine Geschirrspülmaschine mit einer Trocknungseinrichtung bekannt. Während einer Trocknungsphase kann Trocknungsluft über eine Auslassöffnung in einen Spülbehälter eingeleitet werden. Dabei wird der Trocknungsluftstrom durch eine mechanische Bewegung eines Elementes, wie beispielsweise einer Lochscheibe, während der Trocknungsphase mehrfach variiert. Dadurch kann die Trocknung unabhängig von der Beladung intensiviert werden.

Immer dann, wenn eine Medientrenneinrichtung eingesetzt wird, also insbesondere eine Einrichtung, die einen Luftstrom von einem Flüssigkeitsstrom trennt, muss darauf geachtet werden, dass sämtliche Bereiche dieser Medientrenneinrichtung, die mit der Spülflüssigkeit in Kontakt kommen können, auch desinfiziert werden müssen, damit sich in jenen Bereichen keine Keime vermehren und zu einer Rekontamination des Spülgutes führen können. Rückschlagklappen oder Rückschlagventile in einem Trocknungsstrang weisen üblicherweise einen geringen Öffnungsdruck auf. Sie können daher das Eindringen von Feuchtigkeit in den eigentlich trocken zu haltenden Trocknungsstrang nicht vollständig verhindern. Dies gilt insbesondere dann, wenn die Dichtfläche bereits abgenutzt ist. Dadurch bestehen die Gefahr eines Kurzschlusses an Heizelementen innerhalb des Trocknungsstrangs sowie die Gefahr einer Rekontamination durch das Einbringen kontaminierter Trocknungsluft in die Reinigungskammer eines Reinigungs- und Desinfektionsgeräts.

Für die in der Regel gleichzeitig durchgeführte Innen- und Außentrocknung steht bei den aus dem Stand der Technik bekannten Lösungen jeweils nur ein begrenzter Volumenstrom zur Verfügung. Eine Optimierung der Trocknungsleistung entweder der Innentrocknung oder der Außentrocknung ist dabei nicht möglich.

Der vorliegenden Erfindung liegt die Aufgabe zugrunde, ein Reinigungs- und Desinfektionsgerät mit einer gegenüber dem Stand der Technik verbesserten Medientrenneinrichtung bereitzustellen, die eine Optimierung der Trocknungsleistung für die Innentrocknung und die Außentrocknung der zu trocknenden Instrumente ermöglicht und gleichzeitig die Gefahr einer Rekontamination von bereits desinfizierten Gegenständen effektiv reduziert. Darüber hinaus soll ein entsprechendes Trocknungsverfahren angegeben werden.

Diese Aufgabe wird mit einem Reinigungs- und Desinfektionsgerät mit den Merkmalen des Anspruchs 1 gelöst. Ein solches Reinigungs- und Desinfektionsgerät ist zur Reinigung und Desinfektion medizinischer und/oder zahnmedizinischer Instrumente ausgelegt. Es weist eine Medientrenneinrichtung auf, die einen ersten Einlass für Spülflüssigkeit, einen zweiten Einlass für Trocknungsluft sowie einen gemeinsamen Auslass für Spülflüssigkeit und Trocknungsluft aufweist. Durch den gemeinsamen Auslass kann wahlweise Spülflüssigkeit oder Trocknungsluft aus der Medientrenneinrichtung ausströmen. Als Trocknungsluft kann beispielsweise erwärmte trockene Luft eingesetzt werden, wobei die Erwärmung beispielsweise mittels einer Trocknungsheizung durchgeführt werden kann.

Erfindungsgemäß ist vorgesehen, dass das Reinigungs- und Desinfektionsgerät ein Antriebselement aufweist, das dazu dient, ein in der Medientrenneinrichtung beweglich gelagertes Absperrelement zu bewegen. Dabei kann das Absperrelement zumindest in eine erste Position, eine zweite Position und eine dritte Position bewegt werden. In der ersten Position verschließt das Absperrelement den ersten Einlass. In der zweiten Position verschließt das Absperrelement den zweiten Einlass. In der dritten Position verschließt das Absperrelement den gemeinsamen Auslass.

Dabei ist das Antriebselement unabhängig von einer Einrichtung zur Förderung von Spülflüssigkeit oder Trocknungsluft durch die Medientrenneinrichtung (wie etwa einer Pumpe oder einem Gebläse) ausgeführt. Das heißt, das Antriebselement kann vollkommen unabhängig von einem Betriebszustand und einem gerade durch die Medientrenneinrichtung strömenden Fluid angesteuert werden und eine Bewegung des Absperrelements in die gewünschte Position bzw. ein Halten des Absperrelements in der gewünschten Position bewirken. Bei dem Antriebselement handelt es sich insbesondere um einen Motor, wie etwa einen Getriebemotor.

Wenn sich das Absperrelement in seiner ersten Position befindet, kann Trocknungsluft durch den zweiten Einlass in die Medientrenneinrichtung einströmen und durch den gemeinsamen Auslass aus der Medientrenneinrichtung ausströmen. Die Trocknungsluft gelangt dann mit wenig Druckverlust in die Aufbereitungskammer des Reinigungs- und Desinfektionsgeräts und kann eine Außentrocknung der zu trocknenden medizinischen und/oder zahnmedizinischen Instrumente mit hohem Volumenstrom bewirken.

Wenn sich das Absperrelement in seiner zweiten Position befindet, kann Spülflüssigkeit durch den ersten Einlass in die Medientrenneinrichtung einströmen und durch den gemeinsamen Auslass aus der Medientrenneinrichtung ausströmen. Wenn sich das Absperrelement in seiner dritten Position befindet, sind der erste Einlass und der zweite Einlass strömungstechnisch miteinander verbunden. Dann ist es möglich, dass Trocknungsluft durch den zweiten Einlass in die Medientrenneinrichtung einströmt und durch den ersten Einlass aus der Medientrenneinrichtung ausströmt. Daher dient der erste Einlass in einer Variante auch als Auslass für die Trocknungsluft.

Es ist dann auf besonders einfache Weise möglich, die Medientrenneinrichtung wahlweise zur Außentrocknung oder zu Innentrocknung von medizinischen und/oder zahnmedizinischen Instrumenten einzusetzen, die sich im Reinigungs- und Desinfektionsgerät befinden. Zu diesem Zweck ist die Medientrenneinrichtung vorzugsweise derart im Reinigungs- und Desinfektionsgerät angeordnet, dass der gemeinsame Auslass für Spülflüssigkeit und Trocknungsluft in einer Reinigungskammer des Reinigungs- und Desinfektionsgerätes mündet, sodass Trocknungsluft, die durch den gemeinsamen Auslass aus der Medientrenneinrichtung ausströmt, zur Außentrocknung der Instrumente eingesetzt wird.

Da die Leitung, mit der Spülflüssigkeit der Medientrenneinrichtung zugeführt wird, mit den Innenlumina von Instrumenten, die im Reinigungs- und Desinfektionsgerät aufgenommen sind, strömungstechnisch verbunden ist, kann dieser ohnehin vorhandene Strömungspfad verwendet werden, um die Trocknungsluft durch den ersten Einlass aus der Medientrenneinrichtung ausströmen, zu den Innenlumina der Instrumenten hinströmen und durch diese Innenlumina durchströmen zu lassen. Damit die Trocknungsluft tatsächlich zu den Innenlumina der derart aufgenommenen Instrumente gelangt, kann im Strömungspfad der Spülflüssigkeit beispielsweise eine Rückschlagklappe vorgesehen sein, die automatisch geöffnet wird, wenn Spülflüssigkeit zur Medientrenneinrichtung hin strömt, von in die entgegengesetzte Richtung strömender Trocknungsluft jedoch automatisch verschlossen wird.

Das Antriebselement sorgt in einer Variante auch dafür, dass das Absperrelement in der Position, in die es überführt wurde, verbleibt. In einer anderen Variante sorgt das Antriebselement für eine kontinuierliche Bewegung des Absperrelements von einer Position in eine andere Position. So kann beispielsweise eine Rotationsbewegung des Absperrelements erfolgen, um einen fortlaufenden Wechsel von Innentrocknung, Außentrocknung und gemeinsamer Innen- und Außentrocknung der Instrumente zu ermöglichen. Dann wird das Absperrelement nicht in der jeweiligen Position gehalten, sondern gleich nach Erreichen der jeweiligen Position in eine andere Position überführt.

In einer Variante ist es möglich, dass das Absperrelement in einer vierten Position vorliegt, in der es weder den ersten Einlass noch den zweiten Einlass noch den gemeinsamen Auslass verschließt. Dann ist es möglich, dass Trocknungsluft durch den zweiten Einlass in die Medientrenneinrichtung einströmt und sowohl durch den gemeinsamen Auslass als auch durch den als weiteren Auslass für Trocknungsluft dienenden ersten Einlass aus der Medientrenneinrichtung ausströmt. In diesem Betriebsmodus ist es folglich möglich, dass die der Medientrenneinrichtung zugeführte Trocknungsluft sowohl zur Innentrocknung als auch zur Außentrocknung von in dem Reinigungs- und Desinfektionsgerät angeordneten bzw. aufgearbeiteten Instrumenten eingesetzt wird.

In einer Variante ist das Absperrelement drehbar um eine Achse gelagert, wobei insbesondere eine Bewegung (Rotation) um diese Achse über einen Winkelbereich von 360° möglich ist. Dann lassen sich die erste Position, die zweite Position, die dritte Position und gegebenenfalls die vierte Position besonders leicht durch eine entsprechende Rotation des Absperrelementes realisieren.

In einer Variante lässt sich das Absperrelement nicht nur in die erste, zweite, dritte und gegebenenfalls vierte Position überführen, sondern auch in Zwischenpositionen zwischen diesen Positionen. Dabei handelt es sich bei den Zwischenpositionen insbesondere um beliebige Zwischenpositionen zwischen den vorgenannten Positionen. So lässt sich das Absperrelement in dieser Variante mittels des Antriebselements insbesondere in eine Zwischenposition zwischen der ersten Position der zweiten Position oder zwischen der zweiten Position und der dritten Position oder zwischen der dritten Position und der ersten Position überführen. Dabei lässt sich das Absperrelement durch das Antriebselement bei Bedarf (optional) auch in diesem Zwischenpositionen halten. Durch derartige Zwischenpositionen ist es möglich, dass der erste Einlass und/oder der zweite Einlass und/oder der gemeinsame Auslass durch das Absperrelement nicht vollständig verschlossen werden, sondern teilweise geöffnet verbleiben. Dann kann ein verminderter Volumenstrom an Spülflüssigkeit oder Trocknungsluft durch die Medientrenneinrichtung hindurch zum gemeinsamen Auslass bzw. zum weiteren Auslass für Trocknungsluft strömen.

Während eines Desinfektionsschritts wird heiße Spülflüssigkeit durch den ersten Einlass in die Medientrenneinrichtung eingebracht. Dabei kann der Füllstand der Spülflüssigkeit in der Medientrenneinrichtung ansteigen und somit für eine Erhöhung der Temperatur des gesamten Gehäuses der Medientrenneinrichtung sorgen. Dadurch kommt es zu einer effektiven Desinfektion des gesamten Innenraums der Medientrenneinrichtung. Ein Ansteigen der Spülflüssigkeit während des Desinfektionsschritts kann besonders einfach dadurch erreicht werden, dass das Absperrelement in eine Zwischenposition überführt wird, in der es den gemeinsamen Auslass teilweise verschließt. Dann steht ein im Vergleich zum Spülflüssigkeitseinlass verminderter Querschnitt für den Auslass der Spülflüssigkeit aus der Medientrenneinrichtung zur Verfügung, weshalb es zu einem schnellen Anstieg des Spülflüssigkeitsspiegels in der Medientrenneinrichtung kommt.

In einer Variante ist das Absperrelement derart ausgestaltet, dass es den zweiten Einlass für Trocknungsluft zumindest in seiner zweiten Position dampfdicht verschließt. Dies kann über eine entsprechende Ausgestaltung der Außengeometrie des Absperrelementes realisiert werden. Durch einen solchen dampfdichten Verschluss wird die Bildung von Kondensat im Trocknungsstrang vermieden, wodurch eine Kontamination des Trocknungsstrangs verhindert wird.

In einer weiteren Variante ist das Absperrelement derart ausgestaltet, dass es in der Lage ist, den zweiten Einlass für Trocknungsluft auch dann dampfdicht zu verschließen, wenn der gemeinsame Auslass durch das Absperrelement teilweise verschlossen ist.

Um ein dampfdichtes Verschließen des zweiten Einlasses besonders einfach zu ermöglichen, ist ein zusätzliches Verschlusselement zum dampfdichten Verschließen des zweiten Einlasses vorgesehen. Dabei wird dieses weitere Verschlusselement von der Absperreinrichtung in einer Position gehalten, in der es den zweiten Einlass dampfdicht verschließt, wenn sich das Absperrelement in seiner zweiten Position befindet. In einer Variante wird das Verschlusselement auch dann in einer den zweiten Einlass dampfdicht verschließenden Position gehalten, wenn sich das Absperrelement in einer Zwischenposition befindet, in der es auch den gemeinsamen Auslass teilweise verschließt.

Es ist beispielsweise möglich, das Absperrelement mit einer im Querschnitt pilzförmigen Außenkontur mit einem Hut und einem Stil auszugestalten. Dann kann eine gewölbte Oberseite des Huts dafür sorgen, dass ein insbesondere strömungsdichter Verschluss des ersten Einlasses, des zweiten Einlasses oder des gemeinsamen Auslasses erreicht wird, je nachdem, in welcher Position sich das Absperrelement befindet. Es ist bei einer derartigen Ausgestaltung des Absperrelementes auch besonders einfach möglich, dass das Absperrelement in seiner ersten Position mit einer Oberseite seines Huts gegen das weitere Verschlusselement drückt, damit der zweite Einlass dampfdicht verschlossen ist. Auch bei einer anderen Ausgestaltung des Absperrelementes ist ein derartiges Drücken gegen das weitere Verschlusselement möglich, damit dieses Verschlusselement in seiner den zweiten Einlass dampfdicht verschließenden Position gehalten wird.

In einer Variante weist das Reinigungs- und Desinfektionsgerät ein Strömungsbeeinflussungselement auf. Dieses Strömungsbeeinflussungselement ist dabei derart angeordnet, dass zumindest eine Teilmenge der Spülflüssigkeit und Trocknungsluft, die durch den gemeinsamen Auslass aus der Medientrenneinrichtung ausströmt, durch das Strömungsbeeinflussungselement hindurchströmen muss. Dabei wird eine Strömungseigenschaft der Spülflüssigkeit und Trocknungsluft durch das Strömungsbeeinflussungselement beeinflusst. Beispielsweise kann die Richtung der durchströmenden Spülflüssigkeit oder Trocknungsluft zumindest teilweise verändert werden. Es ist auch möglich, dass die durch das Strömungsbeeinflussungselement durchströmende Spülflüssigkeit oder Trocknungsluft kanalisiert wird, sodass sich die Strömungsgeschwindigkeit erhöht. Alternativ oder zusätzlich kann die Laminarität der Strömung erhöht oder erniedrigt werden. Durch ein derartiges Strömungsbeeinflussungselement lassen sich also für die Aufbereitung der medizinischen und/oder zahnmedizinischen Instrumente in dem Reinigungs- und Desinfektionsgerät bzw. für deren Trocknung besonders geeignete Strömungspfade und besonders geeignete Strömungsbedingungen realisieren. Beispielsweise kann eine besonders gleichmäßige Strömung der Trocknungsluft erreicht werden.

Das Strömungsbeeinflussungselement kann beispielsweise als integraler Bestandteil des gemeinsamen Auslasses ausgestaltet sein. In einer Variante ist es in einer dem gemeinsamen Auslass in Strömungsrichtung der Spülflüssigkeit bzw. der Trocknungsluft nachgeordneten Position an der Medientrenneinrichtung angeordnet. Dabei ist es vorzugsweise austauschbar an der Medientrenneinrichtung angeordnet, sodass ein Benutzer je nach Anwendungsfall unterschiedliche Strömungsbeeinflussungselemente an der Medientrenneinrichtung anbringen kann. So lassen sich für verschiedene Beladungen (also verschiedene medizinische und/oder zahnmedizinische Instrumente und Geräte) unterschiedliche Strömungspfade realisieren, die eine besonders effektive Aufbereitung und Trocknung der Instrumente und Geräte ermöglichen. Das Strömungsbeeinflussungselement kann dabei direkt oder indirekt an der Medientrenneinrichtung angeordnet sein. Beispielsweise kann eine indirekte Anordnung dadurch realisiert werden, dass das Strömungsbeeinflussungselement an einer weiteren Komponente wie etwa einem Adapter, der beispielsweise in Form einer Adapterplatte ausgestaltet sein kann, angeordnet ist. Diese weitere Komponente befindet sich dabei in Strömungsrichtung zwischen der Medientrenneinrichtung und der Beladung, also den im Betrieb des Reinigungs- und Desinfektionsgeräts zu behandelnden medizinischen und/oder zahnmedizinischen Instrumenten. Die weitere Komponente ist bei einer indirekten Anordnung des Strömungsbeeinflussungselements in Strömungsrichtung der Spülflüssigkeit und Trocknungsluft folglich zwischen der Medientrenneinrichtung und dem Strömungsbeeinflussungselement angeordnet.

Um einen besonders einfachen Austausch des Strömungsbeeinflussungselementes zu ermöglichen, kann dieses beispielsweise durch einen Steckverschluss, einen Klickverschluss oder einen Bajonettverschluss an der Medientrenneinrichtung bzw. der weiteren Komponente gehalten werden. Dann lässt sich das Strömungsbeeinflussungselement werkzeugfrei von der Medientrenneinrichtung ersatzfrei entfernen oder werkzeugfrei durch ein anderes Strömungsbeeinflussungselement ersetzen.

In einer Variante ist das Strömungsbeeinflussungselement als Medienkupplung ausgestaltet. Dabei ist es in dieser Variante insbesondere dazu vorgesehen und eingerichtet, strömungstechnisch direkt (unmittelbar) oder indirekt (mittelbar) mit einem Innenlumen eines medizinischen und/oder zahnmedizinischen Instruments verbunden zu werden. Beispielsweise kann ein großvolumiger Hohlkörper wie etwa ein Anästhesieschlauch als medizinisches und/oder zahnmedizinisches Instrument direkt mit einem derart als Medienkupplung ausgestalteten Strömungsbeeinflussungselement verbunden werden. Dann wird die aus der Medientrenneinrichtung ausströmende Spülflüssigkeit und Trocknungsluft direkt durch einen derartigen großvolumigen Hohlkörper geleitet. Es ist aber auch denkbar, ein Verteiler- oder Adaptersystem für Instrumente mit dem als Medienkupplung ausgestalteten Strömungsbeeinflussungselement zu verbinden. Anschließend können dann Instrumente auf ein derartiges Verteiler- oder Adaptersystem gesteckt werden. In einem solchen Fall liegt eine mittelbare strömungstechnische Verbindung zwischen dem Strömungsbeeinflussungselement auf der einen Seite und den Instrumenten auf der anderen Seite vor.

In einer Variante weist das Reinigungs- und Desinfektionsgerät mehr als eine Medientrenneinrichtung gemäß den vorherigen Erläuterungen auf, insbesondere mindestens oder genau 2, 3, 4 oder 5 derartige Medientrenneinrichtungen.

In einer Variante ist es vorgesehen, dass jeder Beladungsebene des Reinigungs- und Desinfektionsgerät mindestens eine, insbesondere genau eine dieser Medientrenneinrichtungen zugeordnet ist. Typischerweise weisen Reinigungs- und Desinfektionsgeräte zwei bis drei Beladungsebenen auf, sodass in einem solchen Fall auch eine der Anzahl der Beladungsebenen entsprechende Anzahl von Medientrenneinrichtungen vorgesehen ist.

Dabei ist es in einer Variante vorgesehen, dass die Medientrenneinrichtung (oder zumindest der gemeinsame Auslass) jeweils unter der Beladungsebene, der sie zugeordnet ist, angeordnet ist. Dann kann die durch den gemeinsamen Auslass ausströmende Trocknungsluft von unten auf der Außenseite der in der entsprechenden Beladungsebene angeordneten medizinischen und/oder zahnmedizinischen Instrumente entlanggeführt werden und für eine effektive Außentrocknung dieser Instrumente sorgen. Durch eine derartige Positionierung lassen sich auch weit entfernte Bereiche innerhalb der Aufbereitungskammer des Reinigungs-und Desinfektionsgeräts von der Trocknungsluft erreichen. Außerdem wird die aus dem gemeinsamen Auslass ausströmende Trocknungsluft nicht von fehlerhaft platzierte Beladung am weiteren Strömen durch die Aufbereitungskammer gestört. Dies ist hingegen dann möglich, wenn der gemeinsame Auslass auf einem Niveau neben der entsprechenden Beladung angeordnet ist.

Bei einer Anordnung der Medientrenneinrichtung oder zumindest des gemeinsamen Auslasses unterhalb der zugeordneten Beladungsebene ist es außerdem besonders einfach möglich, dass die Trocknungsluft, die zur Innentrocknung der entsprechenden medizinischen und/oder zahnmedizinischen Instrumente vorgesehen ist, von unten durch ein Innenlumen dieser Instrumente durchgeleitet wird und oben aus den Instrumenten austritt. Dabei treibt die Trocknungsluft in dem Innenlumen befindliche Flüssigkeit nach oben aus.

Die der Erfindung zugrunde liegende Aufgabe wird auch durch ein Verfahren zum Trocknen medizinischer und/oder zahnmedizinischer Instrumente, das die nachfolgend erläuterten Schritte aufweist, gelöst. Dabei sind die medizinischen und/oder zahnmedizinischen Instrumente in einem Reinigungs- und Desinfektionsgerät gemäß den vorherigen Erläuterungen aufgenommen. Insbesondere wurden sie vor dem Trocknungsverfahren in dem Reinigungs- und Desinfektionsgerät aufbereitet.

Das Trocknungsverfahren zeichnet sich dadurch aus, dass Trocknungsluft mittels einer Medientrenneinrichtung, die ein beweglich gelagertes Absperrelement aufweist, durch eine Bewegung des Absperrelementes in verschiedene Richtungen geleitet wird. So kann die Trocknungsluft wahlweise nur zur Außentrocknung, nur zur Innentrocknung oder zur gemeinsamen Innen- und Außentrocknung eingesetzt werden. Das heißt, die Trocknungsluft wird wahlweise nur auf eine Außenseite der Instrumente, nur durch ein Innenlumen der Instrumente oder aber sowohl auf die Außenseite als auch durch das Innenlumen der Instrumente geleitet. Die Bewegung des Absperrelementes erfolgt dabei mittels eines Antriebselementes, das unabhängig von einer Einrichtung zur Förderung von Spülflüssigkeit oder Trocknungsluft durch die Medientrenneinrichtung (wie etwa einer Pumpe oder eines Gebläses) ausgeführt ist.

Um für eine besonders gute äußere und innere Trocknung der Instrumente zu sorgen, wird die Trocknungsluft in einer Variante abwechselnd wiederholt auf die Außenseite der Instrumenten und durch das Innenlumen der Instrumente geleitet. Auf diese Weise findet ein zyklischer Wechsel zwischen einer Außentrocknung und einer Innentrocknung statt.

In einer Variante lassen sich ein erster Anteil der Trocknungsluft, der zur Außentrocknung auf die Außenseite der Instrumente geleitet wird, und ein zweiter Anteil der Trocknungsluft, der zu Innentrocknung durch das Innenlumen der Instrumente geleitet wird, stufenlos einstellen. Dann ist es besonders einfach möglich, eine optimale Abstimmung zwischen Außentrocknung und Innentrocknung vorzunehmen und die Trocknung auf diese Weise im Hinblick auf die Geometrie der konkret zu trocknenden Instrumente anzupassen bzw. zu optimieren.

In einer Variante wird in einem Strömungspfad der Trocknungsluft ein statischer Druck bestimmt bzw. gemessen. Dieser statische Druck wird dann zur Ermittlung einer Art und/oder einer Menge der Instrumente, die im Reinigungs- und Desinfektionsgerät aufgenommen sind und getrocknet werden sollen, verwendet. Diese Ermittlung kann dabei eine schätzende, näherungsweise Ermittlung der tatsächlichen Beladungsart bzw. Beladungsmenge sein. Eine derartige druckabhängige Überwachung und Kontrolle des Trocknungsverfahrens bietet sich insbesondere dann an, wenn nur eine Innentrocknung der zu trocknenden Instrumente durchgeführt wird. Denn dann ist der gemessene statische Druck im Hinblick auf die Beladungsart und/oder Beladungsmenge besonders aussagekräftig.

In einer Variante wird ein statischer Druck in einem Strömungspfad der Trocknungsluft bestimmt bzw. gemessen und zur Steuerung einer Dauer einer Innentrocknung eingesetzt. Das heißt, der gemessene statische Druck bestimmt, wie lange die Trocknungsluft durch das Innenlumen der zu trocknenden Instrumente geleitet wird. Wenn zahlreiche Instrumente in dem Reinigungs- und Desinfektionsgerät aufgenommen sind und getrocknet werden sollen, ist der statische Druck verhältnismäßig gering (beispielsweise weniger als 10 mbar), da durch die Vielzahl von Instrumenten ein großer Strömungsquerschnitt für die Trocknungsluft bereitgestellt wird. Dann ist es erforderlich, eine verlängerte Innentrocknungsphase durchzuführen. Wenn hingegen nur wenige Instrumente von der Trocknungsluft bei der Innentrocknung durchströmt werden, ist der statische Druck entsprechend höher (beispielsweise 10 mbar bis 130 mbar). Dann genügt eine verhältnismäßig kürzere Dauer der Trocknungsphase, um für eine effektive Innentrocknung der Instrumente zu sorgen.

In einer Variante wird ein statischer Solldruck in einem Strömungspfad in der Trocknungsluft vorbestimmt. Ein geeigneter Solldruck liegt im Bereich von 10 mbar bis 100 mbar, insbesondere 20 mbar bis 90 mbar, insbesondere 30 mbar bis 80 mbar, insbesondere 40 mbar bis 70 mbar, insbesondere 50 mbar bis 60 mbar. Dann wird dieser Solldruck verwendet, um ein drehzahlgesteuertes Gebläse zur Förderung der Trocknungsluft anzusteuern. Zusätzlich wird das Gebläse in Abhängigkeit der Beladungsart und/oder der Beladungsmenge gesteuert, um einen auf die tatsächlich zu trocknenden Instrumente optimierten Trocknungsluftvolumenstrom bereitstellen zu können.

In einer Variante erfolgt die Druckmessung zwischen einem Anschluss, an dem die zu trockenenden Instrumente mit einem Luftzufuhrkanal des Reinigungs- und Desinfektionsgeräts verbunden sind, und dem ersten Einlass der Medientrenneinrichtung. Das heißt, die Druckmessung erfolgt nicht in einem solchen Abschnitt des Strömungspfades der Trocknungsluft, der durch die zu trocknenden Instrumente selbst bereitgestellt wird, sondern in einem Abschnitt des Strömungspfades, der durch das Reinigungs- und Desinfektionsgerät bereitgestellt wird.

Sämtliche Varianten des beschriebenen Reinigungs- und Desinfektionsgeräts sind in analoger Weise auf das beschriebene Verfahren zum Trocknen medizinischer und/oder zahnmedizinischer Instrumente übertragbar, und umgekehrt. Dabei ist eine beliebige Kombination der einzelnen erläuterten Varianten möglich.

Details von Aspekten der vorliegenden Erfindung werden nachfolgend im Zusammenhang mit Ausführungsbeispielen und entsprechenden Figuren näher erläutert. Es zeigen:
- Figur 1: eine schematische Ansicht einer Luftweiche;
- Figur 2: eine erste Querschnittsansicht durch die Luftweiche der Figur 1;
- Figur 3A: eine zweite Querschnittsansicht durch die Luftweiche der Figur 1;
- Figur 3B: eine dritte Querschnittsansicht durch die Luftweiche der Figur 1;
- Figur 3C: eine vierte Querschnittsansicht durch die Luftweiche der Figur 1;
- Figur 3D: eine fünfte Querschnittsansicht durch die Luftweiche der Figur 1;
- Figur 4A: eine Querschnittsansicht durch eine Luftweiche mit einem zugeordneten ersten Strahlrichter;
- Figur 4B: eine Querschnittsansicht durch eine Luftweiche mit einem zugeordneten zweiten Strahlrichter und
- Figur 4C: eine Querschnittsansicht durch eine Luftweiche mit einem zugeordneten dritten Strahlrichter;

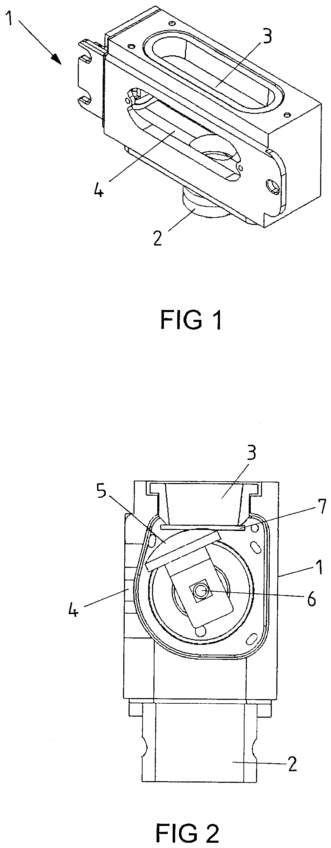

Die Figur 1 zeigt eine Luftweiche 1, die als Medientrenneinrichtung dient. Diese Luftweiche 1 weist an ihrer Unterseite einen ersten Einlass 2 auf, der zum Einleiten von Spülflüssigkeit in die Luftweiche 1 dient. An ihrer Oberseite weist die Luftweiche 1 ein einen zweiten Einlass 3 auf, der zum Einleiten von Trocknungsluft in die Luftweiche 1 dient. Darüber hinaus ist an einer die Oberseite und die Unterseite der Luftweiche 1 verbindenden Seitenwand ein gemeinsamer Auslass 4 ausgebildet, durch den wahlweise Spülflüssigkeit oder Trocknungsluft aus der Luftweiche 1 herausgeleitet werden kann.

Die Figur 2 zeigt eine Querschnittsansicht durch die Luftweiche 1 der Figur 1. Dabei werden in dieser Figur wie auch in den nachfolgenden Figuren die gleichen Bezugszeichen für die bereits aus den zuvor erläuterten Figuren bekannten Elemente verwendet.

Neben dem ersten Einlass 2, den zweiten Einlass 3 und dem gemeinsamen Auslass 4 ist in dieser Querschnittsdarstellung ein um 360° drehbar gelagertes Stellelement 5 zu sehen, das als Absperrelement dient. Dieses Stellelement 5 wird dabei mittels eines nicht dargestellten Motors, der als Antriebselement dient, angetrieben. Der Motor kann das Stellelement 5 dabei um eine in einem zentralen Bereich des Innenraums der Luftweiche 1 verlaufende Achse 6 drehen.

In der Luftweiche 1 ist zudem eine Klappe 7 angeordnet, die als Verschlusselement dient und in der Lage ist, den zweiten Einlass 3, durch den Trocknungsluft in die Luftweiche 1 eingeführt werden kann, dampfdicht zu verschließen. In der in der Figur 2 dargestellten Position drückt eine gewölbte Oberseite eines hutförmig ausgestalteten Abschnitts des insgesamt pilzförmig ausgestalteten Stellelements 5 derart auf die Klappe 7, dass diese den zweiten Einlass 3 dampfdicht verschließt. Dabei weist das Stellelement 5 eine gegenüber der Senkrechten um etwa 45° verdrehte Orientierung auf. In dieser Orientierung verschließt das Stellelement 5 auch einen Teil des dem gemeinsamen Auslass 4 zur Verfügung stehenden Querschnitts. Wenn nun Spülflüssigkeit durch den ersten Einlass 2 in die Luftweiche 1 eingelassen wird, kann diese Spülflüssigkeit die Luftweiche 1 nur langsamer verlassen als sie in die Luftweiche 1 eingeführt wird. Dadurch steigt ein Spülflüssigkeitsspiegel innerhalb der Luftweiche 1 an, sodass das Gehäuse der Luftweiche 1 schnell auf eine geeignete Desinfektionsbedingungen bereitstellende Temperatur gebracht werden kann. Dadurch wird eine thermische Desinfektion der Luftweiche 1 während des Betriebs des Reinigungs- und Desinfektionsgeräts, in dem die Luftweiche 1 eingebaut ist, sichergestellt.

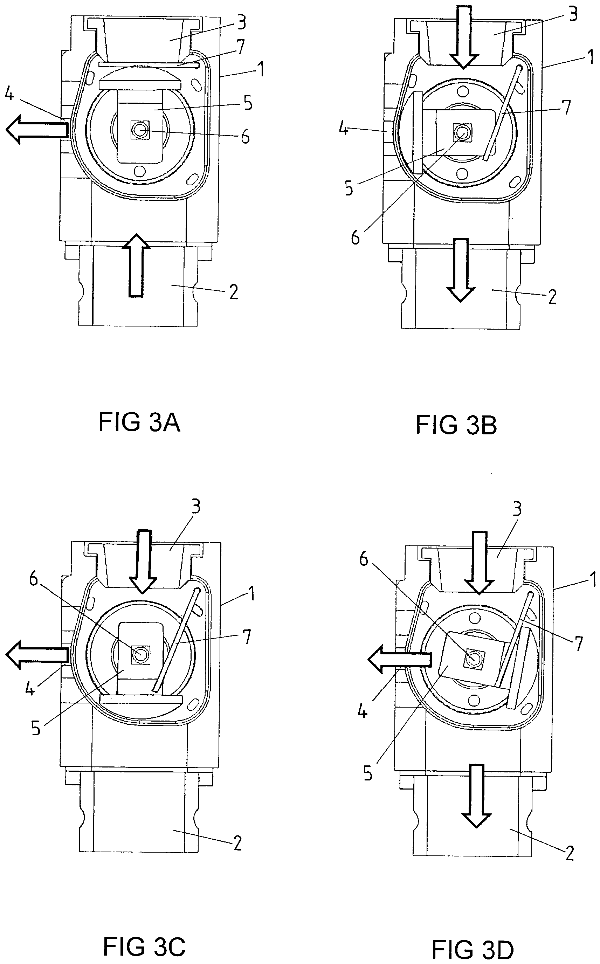

Die Figuren 3A bis 3D zeigen verschiedene Betriebszustände der Luftweiche 1 in Querschnittsdarstellungen.

Die Figur 3A zeigt dabei einen Betriebszustand, der während des Prozessschritts "Desinfizieren" des Reinigungs- und Desinfektionsgeräts von der Luftweiche 1 eingenommen wird. Dabei befindet sich das Stellelement 5 in einer zweiten Position, in der es den zweiten Einlass 3 dampfdicht verschließt. Zu diesem Zweck drückt die gewölbte Oberfläche des hutförmigen Abschnitts des Stellelements 5 gegen die Klappe 7 und verhindert somit ein Eindringen von Spülflüssigkeit oder Dampf in den Trocknungsstrang, durch den die Trocknungsluft in die Luftweiche 1 eingebracht wird. In diesem Betriebszustand sind der erste Einlass 2 und der gemeinsame Auslass 4 geöffnet, sodass Spülflüssigkeit durch die Luftweiche 1 hindurch geleitet werden kann und für eine thermische Desinfektion des Innenraums der Luftweiche 1 sorgen kann. Der Strömungspfad der Spülflüssigkeit ist dabei durch entsprechende Pfeile verdeutlicht.

Die Figur 3B zeigt einen zweiten Betriebszustand der Luftweiche 1, der von der Luftweiche 1 während des Prozessschritts "Innentrocknung" eingenommen wird. In diesem Betriebszustand befindet sich das Stellelement 5 in einer dritten Position, in der es in einer um 90° gegenüber dem in der Figur 3A dargestellten Betriebszustand rotierten Position ausgerichtet ist und den gemeinsamen Auslass 4 verschließt. Dann sind der erste Einlass 2 und der zweite Einlass 3 geöffnet. In diesem Betriebszustand wird Trocknungsluft durch den zweiten Einlass 3 in die Luftweiche 1 eingeführt und kann diese durch den ersten Einlass 2 wieder verlassen. Das heißt, in diesem Betriebszustand dient der erste Einlass 2 zugleich als Auslass für die Trocknungsluft. Der Strömungspfad der Trocknungsluft ist dabei durch entsprechende Pfeile verdeutlicht. Die Trocknungsluft wird dann durch eine Leitung, durch die Spülflüssigkeit zur Luftweiche 1 hingeführt werden kann, von der Luftweiche 1 weggeführt. Diese Leitung ist dabei mit den Innenlumina von Instrumenten strömungstechnisch verbunden, sodass die Trocknungsluft durch die Innenlumina dieser Instrumenten strömt, die in dem Reinigungs- und Desinfektionsgerät, in dem die Luftweiche angeordnet ist, aufbereitet wurden.

Da das Stellelement 5 in dem in der Figur 3B dargestellten Betriebszustand nicht mehr gegen die Klappe 7 drückt, wird diese Klappe 7 von der durch den zweiten Einlass 3 in die Luftweiche 1 einströmenden Trocknungsluft nach unten gedrückt und somit in ihren geöffneten Zustand überführt.

Die Figur 3C zeigt einen weiteren Betriebszustand der Luftweiche 1, der während des Prozessschritts "Außentrocknung" von der Luftweiche 1 eingenommen wird. In diesem Betriebszustand ist das Stellelement 5 gegenüber der in der Figur 3A dargestellten Position um 180° verdreht. Es verschließt dann den ersten Einlass 2, lässt hingegen den zweiten Einlass 3 und den gemeinsamen Auslass 4 geöffnet, sodass diese strömungstechnisch miteinander verbunden sind. Nun kann Trocknungsluft durch den zweiten Einlass 3 in die Luftweiche 1 einströmen und die Luftweiche 1 durch den gemeinsamen Auslass 4 wieder verlassen.

Dabei drückt die Trocknungsluft - wie bei dem in der Figur 3B dargestellten Betriebszustand - die Klappe 7 nach unten und überführt sie somit in ihren geöffneten Zustand. Der Strömungspfad der Trocknungsluft ist dabei durch entsprechende Pfeile verdeutlicht. Der gemeinsame Auslass 4 mündet in eine Aufbereitungskammer des Reinigungs- und Desinfektionsgeräts, in dem die Luftweiche 1 angeordnet ist. Der den gemeinsamen Auslass 4 verlassende Luftstrom trifft somit auf die Außenseiten von medizinischen und/oder zahnmedizinischen Instrumenten, die in der Aufbereitungskammer des Reinigungs- und Desinfektionsgeräts zuvor aufbereitet wurden.

Die Figur 3D zeigt einen weiteren Betriebszustand der Luftweiche 1, in dem das Stellelement 5 um etwa 260° gegenüber der in der Figur 3A dargestellten Position verdreht ist. In dieser Position verschließt das Stellelement 5 weder den ersten Einlass 2 noch den zweiten Einlass 3 noch den gemeinsamen Auslass 4. Vielmehr stehen der erste Einlass 2, der zweite Einlass 3 und der gemeinsame Auslass 4 in diesem Betriebszustand in Strömungsverbindung. Dieser Betriebszustand wird von der Luftweiche 1 insbesondere während des Prozessschritts "Innentrocknung und Außentrocknung" eingenommen. Dann wird Trocknungsluft über den zweiten Einlass 3 in die Luftweiche eingeführt und kann diese durch den gemeinsamen Auslass 4 und den ersten Einlass 2 wieder verlassen. Der Strömungspfad der Trocknungsluft ist dabei durch entsprechende Pfeile verdeutlicht. Auch in diesem Betriebszustand dient der erste Einlass 2 zugleich als (weiterer) Auslass für Trocknungsluft.

Die Trocknungsluft, die die Luftweiche 1 durch den gemeinsamen Auslass 4 verlässt, wird in die Aufbereitungskammer des Reinigungs- und Desinfektionsgeräts geleitet und trifft dann auf die Außenseiten der aufbereiteten medizinischen und/oder zahnmedizinischen Instrumente. Die Trocknungsluft, die die Luftweiche 1 durch den ersten Einlass 2 verlässt, wird über eine Leitung zu den Innenlumina der aufbereiteten medizinischen und/oder zahnmedizinischen Instrumenten geleitet und kann diese Innenlumina anschließend durchströmen. Es erfolgt somit eine gleichzeitige Außentrocknung und Innentrocknung der aufbereiteten Instrumente.

Das Stellelement 5 nimmt die verschiedenen in den Figuren 3A bis 3D gezeigten Positionen dadurch ein, dass es von einem nicht dargestellten Motor um die Achse 6 gedreht wird. Dabei sorgt der Motor dafür, dass das Stellelement sich nicht selbstständig aus einer Position, in die es zuvor überführt wurde, wegbewegen kann. Vielmehr verbleibt es in der Position, in die der Motor es gebracht hat. Mithin ist eine aktive Bewegung des Stellelements 5 erforderlich, um es von einer Position in eine andere Position zu überführen.

Neben den in den Figuren 3A bis 3D dargestellten Positionen des Stellelements 5 sind auch Zwischenpositionen des Stellelements 5 möglich, wie beispielsweise die Zwischenposition, die in der Figur 2 dargestellt ist. Auf diese Weise ist es möglich, einzelne Einlässe bzw. Auslässe der Luftweiche 1 nicht vollständig, sondern nur teilweise zu verschließen. Durch eine stufenlose Bewegung des Stellelements 5 um die Achse 6 herum können dabei den jeweiligen Anforderungen angepasste Positionen des Stellelements 5 realisiert werden, um einen optimierten Strömungsverlauf von Spülflüssigkeit oder Trocknungsluft durch die Luftweiche 1 hindurch ermöglichen. Insbesondere ist es so möglich, eine Außentrocknung mit einem hohen Volumenstrom und eine Innentrocknung mit einem hohen statischen Druck durchzuführen, sodass eine in den Innenlumina der Instrumente befindliche Wassersäule bei aufrechter Anordnung der Instrumenten in der Aufbereitungskammer vollständig aus den in den Innenlumina ausgetrieben werden kann.

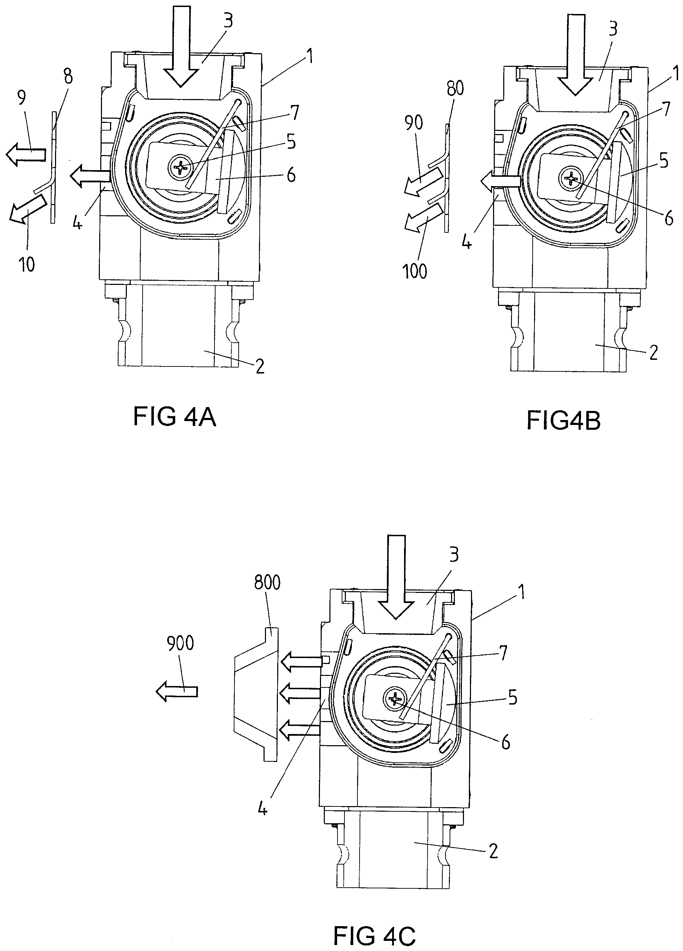

Die Figuren 4A bis 4C zeigen verschiedene Varianten der Luftweiche 1 mit jeweils einem in Strömungsrichtung hinter dem gemeinsamen Auslass 4 angeordneten Strahlrichter 8, 80, 800, der als Strömungsbeeinflussungselement dient. Der Strahlrichter dient dazu, die Richtung und/oder die Geschwindigkeit und/oder die Laminarität der durch ihn hindurchtretenden Strömung zu beeinflussen bzw. zu verändern. Dabei wirken die Strahlrichter 8, 80, 800 gleichermaßen für durch sie hindurchtretende Trocknungsluft wie durch sie hindurchtretende Spülflüssigkeit.

Die Figur 4A zeigt die bereits in den vorherigen Figuren dargestellten Luftweiche 1 mit einem ersten Strahlrichter 8, der dafür sorgt, dass das ein erster Teil 9 des aus dem gemeinsamen Auslass 4 ausströmenden Luftstroms in unveränderter Richtung weiterströmt, während ein zweiter Teil 10 des Luftstroms nach unten abgelenkt wird.

Die Figur 4B zeigt die Luftweiche 1 mit einem zweiten Strahlrichter 80. Dieser zweite Strahlrichter 80 sorgt dafür, dass sowohl ein erster Teil 90 als auch ein zweiter Teil 100 der aus dem gemeinsamen Auslass 4 ausströmenden Trocknungsluft nach unten abgelenkt wird.

Die Figur 4C zeigt schließlich einen dritten Strahlrichter 800, durch den die aus dem gemeinsamen Auslass 4 austretende Trocknungsluft kanalisiert und als kanalisierte Trocknungsluft 900 den dritten Strahlrichter 800 verlässt.

Die Strahlrichter 8, 80, 800 sind dafür vorgesehen und eingerichtet, auf möglichst einfache Weise an der Außenseite der Luftweiche 1 befestigt zu werden, damit sie von einem Benutzer werkzeuglos von der Luftweiche entfernt werden können oder durch einen anderen Strahlrichter, der die durch ihn hindurchtretende Strömung in anderer Weise beeinflusst, ersetzt zu werden. So lassen sich in Abhängigkeit der Beladung des Reinigungs- und Desinfektionsgeräts unterschiedliche Strömungsführungen der Trocknungsluft realisieren, die für eine optimierte Außentrocknung der in der Aufbereitungskammer des Reinigungs- und Desinfektionsgeräts angeordneten Instrumente sorgen.

## Patentansprüche

1. Reinigungs- und Desinfektionsgerät für medizinische und/oder zahnmedizinische Instrumente, mit einer Medientrenneinrichtung (1), wobei die Medientrenneinrichtung (1) einen ersten Einlass (2) für Spülflüssigkeit, einen zweiten Einlass (3) für Trocknungsluft sowie einen gemeinsamen Auslass (4) für Spülflüssigkeit und Trocknungsluft aufweist, durch den hindurch wahlweise Spülflüssigkeit oder Trocknungsluft aus der Medientrenneinrichtung (1) strömen kann,
**dadurch gekennzeichnet,**
**dass** das Reinigungs- und Desinfektionsgerät ein Antriebselement aufweist, mittels dessen ein in der Medientrenneinrichtung (1) beweglich gelagertes Absperrelement (5) in eine erste Position, in der es den ersten Einlass (2) verschließt, in eine zweite Position, in der es den zweiten Einlass (3) verschließt, und in eine dritte Position, in der es den gemeinsamen Auslass (4) verschließt, überführt werden kann, wobei das Antriebselement unabhängig von einer Einrichtung zur Förderung von Spülflüssigkeit oder Trocknungsluft durch die Medientrenneinrichtung (1) ausgeführt ist.

2. Reinigungs- und Desinfektionsgerät nach Anspruch 1, **dadurch gekennzeichnet, dass** das Absperrelement (5) in seiner dritten Position für eine strömungstechnische Verbindung zwischen dem ersten Einlass (2) und dem zweiten Einlass (3) sorgt, so dass der erste Einlass (2) auch als Auslass für Trocknungsluft dient.

3. Reinigungs- und Desinfektionsgerät nach Anspruch 1 oder 2, **dadurch gekennzeichnet, dass** das Absperrelement (5) mittels des Antriebselements in eine Zwischenposition zwischen der ersten Position und der zweiten Position oder zwischen der zweiten Position und der dritten Position oder zwischen der dritten Position und der ersten Position überführt werden und in der Zwischenposition gehalten werden kann, so dass der erste Einlass (2) und/oder der zweite Einlass (3) und/oder der gemeinsame Auslass (4) durch das Absperrelement (5) nicht vollständig verschlossen werden, sondern teilweise geöffnet verbleiben.

4. Reinigungs- und Desinfektionsgerät nach einem der vorherigen Ansprüche, **dadurch gekennzeichnet, dass** die Medientrenneinrichtung (1) ein Verschlusselement (7) zum dampfdichten Verschließen des zweiten Einlasses (3) aufweist, das von der Absperreinrichtung in einer den zweiten Einlass (3) dampfdicht verschlossenen Position gehalten wird, wenn sich das Absperrelement (5) in der zweiten Position befindet.

5. Reinigungs- und Desinfektionsgerät nach einem der vorherigen Ansprüche, **dadurch**
**gekennzeichnet, dass** es ein Strömungsbeeinflussungselement (8, 80, 800) umfassend einen Strahlrichter aufweist,
durch das hindurch zumindest ein Teil der aus der Medientrenneinrichtung (1) austretenden Spülflüssigkeit und Trocknungsluft strömen muss und das eine Strömungseigenschaft der durch das Strömungsbeeinflussungselement (8, 80, 800) strömenden Spülflüssigkeit und Trocknungsluft beeinflusst.

6. Reinigungs- und Desinfektionsgerät nach Anspruch 5, **dadurch gekennzeichnet, dass** das Strömungsbeeinflussungselement (8, 80, 800) austauschbar direkt an der Medientrenneinrichtung (1) oder an oder an einer weiteren Komponente angeordnet ist, die in Strömungsrichtung der Spülflüssigkeit und Trocknungsluft zwischen der Medientrenneinrichtung und dem Strömungsbeeinflussungselement (8, 80, 800) angeordnet ist.

7. Reinigungs- und Desinfektionsgerät nach Anspruch 5 oder 6, **dadurch gekennzeichnet, dass** das Strömungsbeeinflussungselement (8, 80, 800) als Medienkupplung ausgestaltet ist und dazu dient, strömungstechnisch mittelbar oder unmittelbar mit einem Innenlumen eines medizinischen und/oder zahnmedizinischen Instruments verbunden zu werden.

8. Reinigungs- und Desinfektionsgerät nach einem der vorherigen Ansprüche, **dadurch gekennzeichnet, dass** es mindestens zwei der Medientrenneinrichtungen (1) aufweist.

9. Reinigungs- und Desinfektionsgerät nach Anspruch 8, **dadurch gekennzeichnet, dass** jeder Beladungsebene des Reinigungs- und Desinfektionsgeräts eine der Medientrenneinrichtungen (1) zugeordnet ist.

10. Verfahren zum Trocknen medizinischer und/oder zahnmedizinischer Instrumente, die in einem Reinigungs- und Desinfektionsgerät gemäß einem der vorherigen Ansprüche aufgenommen sind,
**dadurch gekennzeichnet,**
**dass** Trocknungsluft mittels einer Medientrenneinrichtung (1), in der sich ein beweglich gelagertes Absperrelement (5) befindet, durch eine Bewegung des Absperrelements (5) mittels eines Antriebselements, welches unabhängig von einer Einrichtung zur Förderung von Spülflüssigkeit oder Trocknungsluft durch die Medientrenneinrichtung (1) ausgeführt ist, wahlweise nur auf eine Außenseite der Instrumente oder nur durch ein Innenlumen der Instrumente oder sowohl auf die Außenseite als auch durch das Innenlumen der Instrumente geleitet wird.

11. Verfahren nach Anspruch 10, **dadurch gekennzeichnet, dass** die Trocknungsluft abwechselnd wiederholt auf die Außenseite der Instrumente und durch das Innenlumen der Instrumente geleitet wird.

12. Verfahren nach Anspruch 10 oder 11, **dadurch gekennzeichnet, dass** ein Anteil der Trocknungsluft, der auf die Außenseite der Instrumente geleitet wird, und ein Anteil der Trocknungsluft, der durch das Innenlumen der Instrumente geleitet wird, stufenlos eingestellt werden können.

13. Verfahren nach einem der Ansprüche 10 bis 12, **dadurch gekennzeichnet, dass** ein statischer Druck in einem Strömungspfad der Trocknungsluft bestimmt und zur Ermittlung einer Art und/oder einer Menge der Instrumente verwendet wird.

14. Verfahren nach einem der Ansprüche 10 bis 13, **dadurch gekennzeichnet, dass** ein statischer Druck in einem Strömungspfad der Trocknungsluft bestimmt und dazu verwendet wird, zu steuern, wie lange die Trocknungsluft durch das Innenlumen der Instrumente geleitet wird.

15. Verfahren nach einem der Ansprüche 10 bis 14, **dadurch gekennzeichnet, dass** ein statischer Solldruck in einem Strömungspfad der Trocknungsluft vorgegeben wird und ein drehzahlgesteuertes Gebläse zur Förderung der Trocknungsluft in Abhängigkeit des Solldrucks und in Abhängigkeit einer Art und/oder einer Menge der Instrumente gesteuert wird.

## Claims

1. Cleaning and disinfecting apparatus for medical and/or dental instruments, with a media separation device (1), the media separation device (1) having a first inlet (2) for rinsing liquid, a second inlet (3) for drying air, and a common outlet (4) for rinsing liquid and drying air, through which common outlet (4) rinsing liquid or drying air can selectively flow out of the media separation device (1),
**characterized in that**
the cleaning and disinfecting apparatus has a drive element, by means of which a shut-off element (5) mounted movably in the media separation device (1) can be transferred to a first position, in which it closes the first inlet (2), to a second position, in which it closes the second inlet (3), and to a third position, in which it closes the common outlet (4), the drive element being designed independently of a device for conveying rinsing liquid or drying air through the media separation device (1).

2. Cleaning and disinfecting apparatus according to Claim 1, **characterized in that** the shut-off element (5), in its third position, ensures a fluidic connection between the first inlet (2) and the second inlet (3), such that the first inlet (2) also serves as an outlet for drying air.

3. Cleaning and disinfecting apparatus according to Claim 1 or 2, **characterized in that** the shut-off element (5) can be transferred by means of the drive element to an intermediate position between the first position and the second position or between the second position and the third position or between the third position and the first position and can be held in the intermediate position, such that the first inlet (2) and/or the second inlet (3) and/or the common outlet (4) are not completely closed by the shut-off element (5) and instead remain partially open.

4. Cleaning and disinfecting apparatus according to one of the preceding claims, **characterized in that** the media separation device (1) has a closure element (7) for closing the second inlet (3) in a vapour-tight manner, which closure element (7) is held by the shut-off device in a position for vapour-tight closure of the second inlet (3) when the shut-off element (5) is located in the second position.

5. Cleaning and disinfecting apparatus according to one of the preceding claims, **characterized in that** it has a flow-influencing element (8, 80, 800) comprising a jet director, through which at least some of the rinsing liquid and drying air emerging from the media separation device (1) has to flow, and which influences a flow property of the rinsing liquid and drying air flowing through the flow-influencing element (8, 80, 800).

6. Cleaning and disinfecting apparatus according to Claim 5, **characterized in that** the flow-influencing element (8, 80, 800) is interchangeably arranged directly on the media separation device (1) or on a further component which, in the flow direction of the rinsing liquid and drying air, is arranged between the media separation device and the flow-influencing element (8, 80, 800).

7. Cleaning and disinfecting apparatus according to Claim 5 or 6, **characterized in that** the flow-influencing element (8, 80, 800) is designed as a media coupling and serves to be fluidically connected indirectly or directly to an inner lumen of a medical and/or dental instrument.

8. Cleaning and disinfecting apparatus according to one of the preceding claims, **characterized in that** it has at least two of the media separation devices (1) .

9. Cleaning and disinfecting apparatus according to Claim 8, **characterized in that** each loading level of the cleaning and disinfecting apparatus is assigned one of the media separation devices (1).

10. Method for drying medical and/or dental instruments received in a cleaning and disinfecting apparatus according to one of the preceding claims,
**characterized in that,**
by means of a media separation device (1), in which a movably mounted shut-off element (5) is located, and by a movement of the shut-off element (5) by means of a drive element, which is designed independently of a device for conveying rinsing liquid or drying air through the media separation device (1), drying air is selectively passed only to an outside of the instruments or only through an inner lumen of the instruments or both to the outside and through the inner lumen of the instruments.

11. Method according to Claim 10, **characterized in that** the drying air is repeatedly passed alternately to the outside of the instruments and through the inner lumen of the instruments.

12. Method according to Claim 10 or 11, **characterized in that** a proportion of the drying air passed to the outside of the instruments and a proportion of the drying air passed through the inner lumen of the instruments can be adjusted in a stepless manner.

13. Method according to one of Claims 10 to 12, **characterized in that** a static pressure in a flow path of the drying air is determined and is used to ascertain a type and/or a quantity of the instruments.

14. Method according to one of Claims 10 to 13, **characterized in that** a static pressure in a flow path of the drying air is determined and is used to control how long the drying air is passed through the inner lumen of the instruments.

15. Method according to one of Claims 10 to 14, **characterized in that** a static target pressure in a flow path of the drying air is predefined, and a speed-controlled fan for conveying the drying air is controlled in accordance with the target pressure and in accordance with a type and/or a quantity of instruments.

## Revendications

1. Appareil de nettoyage et de désinfection pour instruments médicaux et/ou médicodentaires, comprenant un dispositif de séparation de fluides (1), le dispositif de séparation de fluides (1) présentant une première entrée (2) pour un liquide de rinçage, une deuxième entrée (3) pour de l'air de séchage, ainsi qu'une sortie commune (4) pour le liquide de rinçage et l'air de séchage à travers laquelle le liquide de rinçage ou l'air de séchage peut sortir sélectivement du dispositif de séparation de fluides (1),
**caractérisé en ce que** l'appareil de nettoyage et de désinfection présente un élément d'entraînement au moyen duquel un élément de fermeture (5) monté de manière mobile dans le dispositif de séparation de fluides (1) peut être transféré dans une première position dans laquelle il ferme la première entrée (2), dans une deuxième position dans laquelle il ferme la deuxième entrée (3), et dans une troisième position dans laquelle il ferme la sortie commune (4), l'élément d'entraînement étant réalisé indépendamment d'un dispositif de transport du liquide de rinçage ou l'air de séchage à travers le dispositif de séparation de fluides (1).

2. Appareil de nettoyage et de désinfection selon la revendication 1, **caractérisé en ce que** l'élément de fermeture (5) dans sa troisième position assure une communication fluidique entre la première entrée (2) et la deuxième entrée (3) de sorte que la première entrée (2) sert également de sortie à l'air de séchage.

3. Appareil de nettoyage et de désinfection selon la revendication 1 ou 2, **caractérisé en ce qu'**au moyen de l'élément d'entraînement, l'élément de fermeture (5) peut être transféré dans une position intermédiaire entre la première position et la deuxième position ou entre la deuxième position et la troisième position ou entre la troisième position et la première position et peut être maintenu dans la position intermédiaire de sorte que la première entrée (2) et/ou la deuxième entrée (3) et/ou la sortie commune (4) ne sont pas entièrement fermées par l'élément de fermeture (5) mais restent partiellement ouvertes.

4. Appareil de nettoyage et de désinfection selon l'une quelconque des revendications précédentes, **caractérisé en ce que** le dispositif de séparation de fluides (1) présente un élément d'obturation (7) pour l'obturation étanche à la vapeur de la deuxième entrée (3) qui est maintenu par le dispositif de fermeture dans une position obturant la deuxième entrée (3) de manière étanche à la vapeur si l'élément de fermeture (5) se trouve dans la deuxième position.

5. Appareil de nettoyage et de désinfection selon l'une quelconque des revendications précédentes, **caractérisé en ce qu'**il présente un élément influençant l'écoulement (8, 80, 800) qui comprend un redresseur de jet à travers lequel au moins une partie du liquide de rinçage et de l'air de séchage sortant du dispositif de séparation de fluides (1) doit passer, et qui influence une propriété d'écoulement du liquide de rinçage et de l'air de séchage passant à travers l'élément influençant l'écoulement (8, 80, 800).

6. Appareil de nettoyage et de désinfection selon la revendication 5, **caractérisé en ce que** l'élément influençant l'écoulement (8, 80, 800) est disposé de manière remplaçable directement sur le dispositif de séparation de fluides (1) ou sur un autre composant qui est disposé dans la direction d'écoulement du liquide de rinçage et de l'air de séchage entre le dispositif de séparation de fluides et l'élément influençant l'écoulement (8, 80, 800).

7. Appareil de nettoyage et de désinfection selon la revendication 5 ou 6, **caractérisé en ce que** l'élément influençant l'écoulement (8, 80, 800) est configuré sous forme de couplage de fluides et sert à la communication fluidique directe ou indirecte avec une lumière interne d'un instrument médical et/ou médicodentaire.

8. Appareil de nettoyage et de désinfection selon l'une quelconque des revendications précédentes, **caractérisé en ce qu'**il présente au moins deux des dispositifs de séparation de fluides (1).

9. Appareil de nettoyage et de désinfection selon la revendication 8, **caractérisé en ce que** l'un des dispositifs de séparation de fluides (1) est associé à chaque plan d'alimentation de l'appareil de nettoyage et de désinfection.

10. Procédé de séchage d'instruments médicaux et/ou médicodentaires qui sont reçus dans un appareil de nettoyage et de désinfection selon l'une quelconque des revendications précédentes,
**caractérisé en ce qu'**au moyen d'un dispositif de séparation de fluides (1) dans lequel se trouve un élément de fermeture (5) monté de manière mobile, par un mouvement de l'élément de fermeture (5) au moyen d'un élément d'entraînement qui est réalisé indépendamment d'un dispositif de transport du liquide de rinçage ou l'air de séchage à travers le dispositif de séparation de fluides (1), l'air de séchage est guidé sélectivement uniquement sur un côté extérieur des instruments ou uniquement à travers une lumière interne des instruments ou à la fois sur le côté extérieur et à travers la lumière interne des instruments.

11. Procédé selon la revendication 10, **caractérisé en ce que** l'air de séchage est conduit en alternance à plusieurs reprises vers le côté extérieur des instruments et à travers la lumière interne des instruments.

12. Procédé selon la revendication 10 ou 11, **caractérisé en ce qu'**une fraction de l'air de séchage qui est conduite vers le côté extérieur des instruments, et une fraction de l'air de séchage qui est conduite à travers la lumière interne des instruments peuvent être réglées en continu.

13. Procédé selon l'une quelconque des revendications 10 à 12, **caractérisé en ce qu'**une pression statique sur un trajet d'écoulement de l'air de séchage est déterminée et utilisée pour établir un type et/ou une quantité des instruments.

14. Procédé selon l'une quelconque des revendications 10 à 13, **caractérisé en ce qu'**une pression statique sur un trajet d'écoulement de l'air de séchage est déterminée et utilisée pour commander pendant combien de temps l'air de séchage est conduit à travers la lumière interne des instruments.

15. Procédé selon l'une quelconque des revendications 10 à 14, **caractérisé en ce qu'**une pression statique de consigne sur un trajet d'écoulement de l'air de séchage est prédéfinie, et un ventilateur commandé en vitesse de rotation est commandé pour débiter l'air de séchage en fonction de la pression de consigne et en fonction d'un type et/ou d'une quantité des instruments.
